# EUROPÄISCHE PATENTSCHRIFT

(11) **EP 1 677 742 B1**
(45) Veröffentlichungstag und Bekanntmachung des Hinweises auf die Patenterteilung: **24.01.2007**
(21) Anmeldenummer: 04764993.4
(22) Anmeldetag: 09.09.2004
(51) Int. Cl.: A61K 8/02, A61K 8/44, A61K 8/73

(54) **PULVERFÖRMIGE ZUBEREITUNGEN, ENTHALTEND DIETHYLAMINO-HYDROXYBENZOYL-HEXYL-BENZOAT**
POWDERY PREPARATIONS CONTAINING DIETHYLAMINO-HYDROXYBENZOYL-HEXYL-BENZOATE
PREPARATIONS PULVERULENTES CONTENANT DU DIETHYLAMINO-HYDROXYBENZOYL-HEXYL-BENZOATE

(30) Priorität: 15.09.2003 DE 10342860
(43) Veröffentlichungstag der Anmeldung: 12.07.2006
(73) Patentinhaber: BASF Aktiengesellschaft, 67056 Ludwigshafen (DE)
(72) Erfinder: GOEDEL, Werner, 89081 Ulm (DE); BLANZ, Birgit, 67435 Neustadt (DE); AUWETER, Helmut, 67117 Limburgerhof (DE); ANDRE, Valerie, 67063 Ludwigshafen (DE); SCHAPER, Michael, 67065 Ludwigshafen (DE); HABICH, Andreas, 67346 Speyer (DE)
(86) Internationale Anmeldenummer: PCT/EP2004/010057
(87) Internationale Veröffentlichungsnummer: WO 2005/025529

(56) Entgegenhaltungen:
- EP-A- 0 925 777
- EP-A- 1 127 567
- EP-A- 1 352 639
- WO-A-98/19652

## Beschreibung

Die Erfindung betrifft pulverförmiger Zubereitungen, enthaltend Diethylamino-hydroxybenzoyl-hexyl-benzoat, deren Herstellung sowie deren Verwendung als photostabile Lichtschutzmittel.

Die Qualität bzw. Lebensdauer vieler organischer Materialien, beispielsweise Kunststoffe und Lacke aber auch pharmazeutische und kosmetische Zubereitungen kann durch Einwirkung von Licht, insbesondere durch UV-Strahlen negativ beeinträchtigt werden. Diese Qualitätseinbußen zeigen sich bei Kunststoffen und Lacken häufig in Vergilbung, Verfärbung, Rissbildung oder Versprödung des Materials. Im Falle pharmazeutischer und kosmetischer Zubereitungen kann es durch den Einfluss von UV-Strahlen zum Abbau der in den Formulierungen enthaltenen Wirkstoffen kommen.

Die schädigende Wirkung des ultravioletten Teils der Sonnenstrahlung auf die Haut oder Haare, die im weitesten Sinne auch ein organisches Material darstellen, ist ebenfalls ein Problem, das immer mehr an Bedeutung gewinnt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, auf der Haut ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich u.a. um Triazinderivate, Derivate des 3-Benzylidencamphers, der 4-Aminobenzoesäure, der Zimtsäure, der Salicylsäure, des Benzophenons sowie des 2-Phenylbenzimidazols handelt.

Auch für den Bereich zwischen etwa 320 nm und etwa 400 nm, den sogenannten UVA-Bereich, ist es wichtig, Filtersubstanzen zur Verfügung zu haben, da dessen Strahlen Reaktionen bei lichtempfindlicher Haut hervorrufen können. Es ist erwiesen, dass UVA-Strahlung zu einer Schädigung der elastischen und kollagenen Fasern des Bindegewebes führt, was die Haut vorzeitig altern lässt, und dass sie als Ursache zahlreicher phototoxischer und photoallergischer Reaktionen zu sehen ist. Der schädigende Einfluss der UVB-Strahlung kann durch UVA-Strahlung verstärkt werden.

Zum Schutz gegen UVA-Strahlen werden Derivate des Dibenzoylmethans verwendet, deren Photostabilität jedoch nicht in ausreichendem Maße gegeben ist (Int. J. Cosm. Science 10, 53 (1988)).

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, dass auch ionische Spezies bei UV-Exposition entstehen, welche dann ihrerseits oxidativ in die biochemischen Prozesse einzugreifen vermögen.

Ein anwendungstechnischer Nachteil vieler UV-Filter ist deren schlechte Löslichkeit in Wasser und/oder in natürlichen und synthetischen Ölen, z.B. in Silikonölen und in Fettsäuretriglyceriden, wodurch deren Verwendung beispielsweise in kosmetischen Formulierungen häufig eingeschränkt ist.

Ein weiterer Nachteil bei der Applikation einiger Lichtschutzmittel ist das Auftreten von Hautirritationen und Allergien aufgrund von zu hoher Hautdurchlässigkeit.

Zur Verbesserung der Formulierbarkeit unlöslicher bzw. nur schwerlöslicher UV-Absorber sind bereits zahlreiche Verfahren veröffentlicht worden.

So wird in GB-A-2 303 549 ein Mahlverfahren zur Herstellung von mikronisierten unlöslichen organischen UV-Absorbem in Gegenwart von Alkylpolyglykosiden beschrieben. Die so erhaltenen Mikronisate können in kosmetischen Lichtschutzzubereitungen eingearbeitet werden.

GB-A-2 286 774 beinhaltet ebenfalls ein Mahlverfahren zur Mikronisierung unlöslicher organischer UV-Absorber.

EP-A-1 127 567 beschreibt wässrige Dispersionen schwer wasserlöslicher oder wasserunlöslicher organischer UV-Filtersubstanzen und daraus hergestellte Trockenpulver, dadurch gekennzeichnet, dass sie mindestens eine schwer wasserlösliche oder wasserunlösliche organische UV-Filtersubstanz als kolloid-disperse Phase in amorpher oder teilamorpher Form enthalten. Die Verwendung der in dieser Schrift genannten Schutzkolloide - insbesondere Gelatine oder Casein bzw. Caseinat - führt zu pulverförmigen Produkten, deren Kaltwasserlöslichkeit unbefriedigend ist. Ferner können Gelatine und Casein in kosmetischen Formulierungen Hautallergien hervorrufen.

Es war nun Aufgabe der vorliegenden Erfindung, ein Verfahren zur Herstellung von Benzoylbenzoat-haltigen Lichtschutzmittel-Formulierungen bereitzustellen, die einen wirkungsvollen Schutz für organisches Material insbesondere für die menschliche Haut und/oder menschliche Haare vor UV-Strahlen mit sich bringen, die gut hautverträglich sind und die sich sowohl in lipophilen als auch insbesondere in wässrigen Systemen gut einarbeiten lassen.

Diese Aufgabe wurde gelöst durch ein Verfahren zur Herstellung pulverförmiger Zubereitungen, enthaltend Diethylamino-hydroxybenzoyl-hexyl-benzoat der Formel I 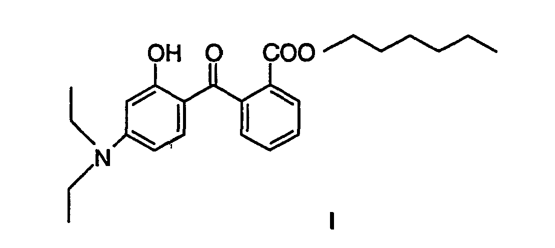 durch
a) Dispergieren des Benzoylbenzoats I in einer wässrigen molekulardispersen oder kolloiddispersen Lösung eines Schutzkolloids und
b) Überführung der gebildeten Dispersion in ein Trockenpulver durch Abtrennung des Wassers und gegebenenfalls zusätzlich verwendeter Lösungsmittel und Trocknung,
dadurch gekennzeichnet, dass man als Schutzkolloid im Verfahrensschritt a) modifizierte Stärke verwendet.

Diethylamino-hydroxybenzoyl-hexyl-benzoat der Formel I wird von der BASF Aktiengesellschaft unter der Warenbezeichnung Uvinul^{®} A Plus als UVA-Filter vermarktet. Uvinul^{®} A Plus zeichnet sich u.a. durch hohe Photostabilität und gute UV-Absorptionseigenschaften mit einem hohen Extinktionskoeffizienten von 940 bei 354 nm aus.

Unter der Bezeichnung wässrige Dispersionen sind im Rahmen der vorliegenden Erfindung sowohl wässrige Suspensionen als auch Emulsionen zu verstehen. Bevorzugt sind wässrige Suspensionen zu nennen, bei denen die dispergierte Phase das Benzoylbenzoat I als nanopartikuläre Teilchen enthält.

Der Begriff modifizierte Stärke umfasst im Rahmen der vorliegenden Erfindung bevorzugt Ester der Stärke mit organischen Säuren, z.B. mit Essigsäure und höheren Fettsäuren (C₆-C₂₆), sowie mit Bernsteinsäure, Adipinsäure und Citronensäure. Die Stärke kann dabei u.a. aus Mais, Kartoffeln oder Weizen gewonnen werden. Als besonders bevorzugte modifizierte Stärke sei Octenyl-succinat-Stärke genannt, das unter der Warenbezeichnung HiCap^{®} von National Starch oder EmCap^{®} von Cerestar vermarktet wird.

Eine bevorzugte Variante des erfindungsgemäßen Verfahrens ist dadurch gekennzeichnet, dass das Dispergieren in der Stufe a) folgende Schritte umfasst:
a₁) Lösen des Benzoylbenzoats I in einem oder mehreren mit Wasser mischbaren, organischen Lösungsmittel(n) oder in einer Mischung aus Wasser und einem oder mehreren mit Wasser mischbaren, organischen Lösungsmittel(n) oder
a₂) Lösen des Benzoylbenzoats I in einem oder mehreren mit Wasser nicht mischbaren, organischen Lösungsmittel(n) und
a₃) Mischen der nach a₁) oder a₂) erhaltenen Lösung mit einer wässrigen molekulardispersen oder kolloiddispersen Lösung von modifizierter Stärke, wobei die hydrophobe Phase des Benzoylbenzoats I als nanodisperse Phase entsteht.

Je nach Art der verwendeten Lösungsmittel kann es sich bei der nanodispersen Phase im Schritt a₃) um feste Nanopartikel [Suspension; erhältlich durch Kombination von a₁) und a₃)] oder um Nanotröpfchen (Emulsion; erhältlich durch Kombination von a₂) und a₃)] handeln.

Die in der Stufe a₁) verwendeten wassermischbaren Lösungsmittel sind vor allem wassermischbare, thermisch stabile, flüchtige, nur Kohlenstoff, Wasserstoff und Sauerstoff enthaltende Lösungsmittel wie Alkohole, Ether, Ester, Ketone und Acetale zu nennen. Zweckmäßig verwendet man solche Lösungsmittel, die mindestens zu 10 % wassermischbar sind, einen Siedepunkt unter 200°C, bevorzugt unter 100°C aufweisen und/oder weniger als 10 Kohlenstoffe haben. Besonders bevorzugt sind Methanol, Ethanol, n-Propanol, Isopropanol, 1,2-Butandiol-1-methylether, 1,2-Propandiol-1-n-propylether, Tetrahydrofuran oder Aceton oder Mischungen davon, ganz besonders bevorzugt werden isopropanol oder Aceton verwendet.

Der Begriff "ein mit Wasser nicht mischbares organisches Lösungsmittel" steht im Sinne der vorliegenden Erfindung für ein organisches Lösungsmittel mit einer Wasserlöslichkeit bei Normaldruck von weniger als 10 %. Als mögliche Lösungsmittel kommen dabei u.a. halogenierte aliphatische Kohlenwasserstoffe, wie z.B. Methylenchlorid, Chloroform und Tetrachlorkohlenstoff, Carbonsäureester wie Diethylcarbonat, Ethylformiat, Methyl-, Ethyl- oder Isopropylacetat sowie Ether wie Methyl-tert. butylether in Frage. Bevorzugte, mit Wasser nicht mischbare organische Lösungsmittel sind die folgenden Verbindungen aus der Gruppe, bestehend aus Dimethylcarbonat, Propylencarbonat, Ethylformiat, Ethylacetat, Isopropylacetat und Methyl-tert. butylether.

Die Herstellung des Trockenpulvers im Verfahrensschritt b) kann dabei u.a. durch Sprühtrocknung, Sprühkühlung, Gefriertrocknung sowie durch Trocknung im Wirbelbett, Konvektionstrocknung oder Kontakttrocknung erfolgen, wobei die Trocknung auch in Gegenwart eines Überzugsmaterials (Puderungsmittels) durchgeführt werden kann. Als Überzugsmittel eignen sich u.a. Maisstärke, Kieselsäure oder auch Tricalciumphosphat.

Bei der Lyophilisation der erfindungsgemäßen Nanopartikel können kryoprotektive Substanzen wie z.B. Trehalose oder Polyvinylpyrrolidone zugesetzt werden.

Besonders bevorzugt ist eine Ausführungsform des erfindungsgemäßen Verfahrens, bei der man
a₁) das Benzoylbenzoat I in Aceton oder Isopropanol oder einer Mischung aus Wasser und Aceton oder Wasser und Isopropanol bei Temperaturen im Bereich von 50 bis 240°C löst,
a₃) die erhaltene Lösung mit einer wässrigen molekulardispersen oder kolloiddispersen Lösung von modifizierter Stärke, insbesondere Octenyl-succinat-Stärke bei Temperaturen im Bereich von 25 bis 120°C mischt und
b) die gebildete Suspension nach Abtrennung des organischen Lösungsmittels sprühtrocknet.

Die Herstellung der o.g. Trockenpulver erfolgt vorteilhafterweise so, dass man das Benzoylbenzoat der Formel I in Aceton oder Isopropanol oder einer Mischung aus Wasser und Aceton oder Wasser und Isopropanol bei Temperaturen im Bereich von 50°C bis 240°C, insbesondere 100°C bis 200°C, besonders bevorzugt im Bereich von 105°C bis 180°C löst.

Zur raschen Herstellung der molekulardispersen Lösung kann die Anwendung von erhöhtem Druck, z.B. im Bereich von 20 bar bis 200 bar, vorzugsweise 30 bis 100 bar, vorteilhaft sein.

Die so erhaltene molekulardisperse Lösung versetzt man anschließend direkt mit der gegebenenfalls gekühlten wässrigen molekulardispersen oder kolloiddispersen Lösung der modifizierten Stärke, insbesondere Octenyl-succinat-Stärke in der Weise, dass sich eine Mischungstemperatur von etwa 25°C bis 120°C, bevorzugt 40°C bis 80°C, besonders bevorzugt 45°C bis 70°C einstellt.

Dabei wird die Lösungsmittelkomponente in die wässrige Phase überführt und die hydrophobe Phase des Benzoylbenzoats entsteht als nanodisperse Phase.

Das Mischen im Schritt a₃) kann in der Art erfolgen, dass man die Benzoylbenzoat-haltige Lösung vorlegt und die wässrige Lösung von modifizierter Stärke zudosiert oder umgekehrt oder dass man bevorzugt beide Lösungen gleichzeitig und kontinuierlich in eine Mischkammer dosiert.

Hinsichtlich einer näheren Verfahrens- und Apparatebeschreibung zur oben genannten Dispergierung wird an dieser Stelle auf EP-B-0 065 193 Bezug genommen.

Zur Erhöhung der mechanischen Stabilität des Endproduktes kann es in einigen Fällen zweckmäßig sein, dem Kolloid einen weiteren Veichmacher zuzusetzen, wie Zucker oder Zuckeralkohole, z.B. Saccharose, Glucose, Glucosesirup, Dextrin, Invertzucker, Sorbit, Mannit oder Glycerin.

Zur Erhöhung der Stabilität des Wirkstoffes gegen oxidativen Abbau kann es ebenfalls zweckmäßig sein, Stabilisatoren wie α-Tocopherol, t-Butyl-hydroxy-toluol, t-Butylhydroxyanisol, Ascorbinsäure oder Ethoxyquin zuzusetzen. Sie können entweder der wässrigen oder der Lösungsmittel-Phase zugesetzt werden, vorzugsweise werden sie jedoch gemeinsam mit dem Benzoylbenzoat I in der Lösungsmittel-Phase gelöst.

Weiterhin können die Lichtschutzmittel-Formulierungen niedermolekulare oberflächenaktive Verbindungen (Emulgatoren) in einer Konzentration von 0,01 bis 70 Gew.-%, vorzugsweise 0,1 bis 50 Gew.-%, besonders bevorzugt 0,5 bis 20 Gew.-%, bezogen auf die Trockenmasse der Lichtschutzmittel-Formulierung enthalten. Als solche eignen sich vor allem amphiphile Verbindungen oder Gemische solcher Verbindungen. Grundsätzlich kommen alle Tenside mit einem HLB-Wert von 5 bis 20 in Betracht. Als entsprechende oberflächenaktive Substanzen kommen beispielsweise in Betracht: Ester langkettiger Fettsäuren mit Ascorbinsäure, Mono- und Diglyceride von Fettsäuren und deren Oxymethylierungsprodukte, Ester von Monofettsäureglyceriden mit Essigsäure, Zitronensäure, Milchsäure oder Diacetylweinsäure, Polyglycerinfettsäureester wie z.B. das Monostearat des Triglycerins, Sorbitanfettsäureester, Propylenglykolfettsäureester und Lecitin, Bevorzugt wird Ascorbylpalmitat eingesetzt.

Zur Erhöhung der Stabilität des Wirkstoffes gegen mikrobiellem Abbau kann es zweckmäßig sein, der Zubereitung Konservierungsmittel wie z.B. Methyl-4-hydroxybenzoat, Propyl-4-hydroxybenzoat, Sorbinsäure oder Benzoesäure oder deren Salze zuzusetzen.

Erfindungsgemäß lassen sich damit Trockenpulver erhalten, die ihre in der Primärdispersion gewonnenen Eigenschaften nicht mehr verlieren. Das heißt, dass der amorphe bzw. teilkristalline Charakter der UV-Filtersubstanz erhalten bleibt. Es ist weiterhin eine erfindungsgemäße Eigenschaft, dass diese Pulver beim erneuten Redispergieren mit einer Abweichung von 20 % bevorzugt < 15 % die gleiche Partikeigrößenverteilung zeigen, die sie als Primärdispersion besaßen.

Eine weitere bevorzugte Ausführungsform des o.g Verfahrens ist dadurch gekennzeichnet, dass man die im Verfahrensschritt a) hergestellte Suspension vor der Überführung in ein Trockenpulver mahlt.

Das Mahlverfahren erfolgt dabei vorzugsweise in der Form, dass man das Benzoylbenzoat I in kristalliner Form in einer wässrigen molekulardispersen oder kolloiddispersen Lösung von modifizierter Stärke suspendiert und durch Mahlung auf die gewünschte Partikelgröße zerkleinert.

Die Mahlung kann dabei in an sich bekannter Weise z.B. mit einer Kugelmühle durchgeführt werden. Dabei wird je nach verwendetem Mühlentyp so lange gemahlen, bis die Teilchen eine über Fraunhofer Beugung ermittelte mittlere Partikelgröße D[4,3] von 0,01 bis 100 µm, bevorzugt 0,02 bis 50 µm, besonders bevorzugt 0,05 bis 20 µm, ganz besonders bevorzugt 0,05 bis 5 µm, insbesondere 0,1 bis 1 µm aufweisen. Der Begriff D[4,3] bezeichnet den volumengewichteten mittleren Durchmesser (siehe Handbuch zu Malvern Mastersizer S, Malvern Instruments Ltd., UK).

Durch Erhitzen der wässrigen Suspension nach dem Mahlprozess auf eine Temperatur oberhalb des Schmelzpunktes des Benzoylbenzoats I und anschließende Sprühtrocknung der "Schmelzemulsion" ist es möglich, den amorphen Anteil des Benzoylbenzoats im resultierenden Trockenpulver zu erhöhen. Einzelheiten zu der Mahlung von Wirkstoffen in wässrigen Schutzkolloidlösungen finden sich in EP-B-0 498 824 und EP-B-0 684 973.

Gegenstand der Erfindung sind auch Benzoylbenzoat-haltige pulverförmige Zubereitungen, erhältlich nach den oben genannten Verfahren.

Die neuen Lichtschutzmittel-Formulierungen sind dadurch charakterisiert, dass sie das Benzoylbenzoat der Formel I enthalten, dessen amorpher Anteil im Bereich größer 10 %, bevorzugt größer 30 %, besonders bevorzugt im Bereich von 50 bis 100 %, ganz besonders bevorzugt im Bereich von 75 bis 99 % liegt. Die Bestimmung der Kristallinitätsrate des Benzoylbenzoats I kann dabei beispielsweise durch Röntgenbeugungsmessungen erfolgen.

Der Gehalt an Benzoylbenzoat der Formel I in den erfindungsgemäßen Lichtschutzmittel-Formulierungen liegt im Bereich von 0,1 bis 70 Gew.-%, bevorzugt im Bereich von 2 bis 40 Gew.-%, besonders bevorzugt im Bereich von 3 bis 30 Gew.-%, ganz besonders bevorzugt im Bereich von 5 bis 25 Gew.-%, bezogen auf die Trockenmasse der Formulierungen.

Die mittlere Teilchengröße D[4,3] der nanopartikulären Teilchen in der wässrigen Dispersion liegt je nach Art der Formulierungsmethode im Bereich von 0,01 bis 100 µm, bevorzugt im Bereich von 0,02 bis 50 µm, besonders bevorzugt im Bereich von 0,05 bis 20 µm, ganz besonders bevorzugt im Bereich von 0,05 bis 5 µm, insbesondere 0,1 bis 1 µm.

Während gemahlene UV-Filtersubstanzen bei der Einarbeitung in Hautcremes verstärkt zu Teilchengrößenwachstum neigen, das zu einer Verschlechterung des Sonnenschutzfaktors und zu einem unangenehmen Hautgefühl führen kann, zeigen die erfindungsgemäßen Trockenpulver aufgrund ihrer Matrix und Schutzkolloidstruktur derartige Tendenzen nicht.

Die erfindungsgemäßen Formulierungen - Dispersionen und daraus hergestellte Trockenpulver - eignen sich in hervorragender Weise zum Stabilisieren von organischem Material u.a. gegen die Einwirkung von Licht, Sauerstoff und Wärme. Sie werden dem zu stabilisierenden organischen Material in einer Konzentration von 0,01 bis 10 Gew.-%, bevorzugt 0,01 bis 5 Gew.-%, besonders bevorzugt von 0,02 bis 2 Gew.%, bezogen auf das organische Material, vor, während oder nach seiner Herstellung zugesetzt.

Unter organischem Material sind beispielsweise photographische Aufzeichnungsmaterialien, z.B. photographische Emulsionen oder Vorprodukte für Kunststoffe und Lacke, insbesondere jedoch Kunststoffe und Lacke selbst zu verstehen.

Unter organischem Material sind aber auch kosmetische Zubereitungen wie beispielsweise Cremes, Lotionen, Gele, Lippenstifte zu verstehen.

Gegenstand der vorliegenden Erfindung ist außerdem ein gegen die Einwirkung von Licht, Sauerstoff und Wärme stabilisiertes organisches Material, insbesondere Kunststoffe und Lacke, enthaltend 0,01 bis 10 Gew.-%, bevorzugt 0,01 bis 5 Gew.-%, besonders bevorzugt von 0,02 bis 2 Gew.-%, bezogen auf die Gesamtmenge des organischen Materials, des Benzoylbenzoats I in Form der erfindungsgemäßen Formulierungen.

Zur Vermischung der erfindungsgemäßen Formulierungen vor allem mit Kunststoffen können alle bekannten Vorrichtungen und Methoden zum Einmischen von Stabilisierungsmitteln oder anderen Zusätzen in Polymere angewandt werden.

Das durch die erfindungsgemäßen Formulierungen stabilisierte organische Material kann gegebenenfalls noch weitere Additive enthalten, z.B. Antioxidantien, Lichtstabilisierungsmittel, Metalldesaktivatoren, antistatische Mittel, flammhemmende Mittel, Pigmente und Füllstoffe.

Antioxidantien und Lichtstabilisatoren, die neben den erfindungsgemäßen Formulierungen zugesetzt werden können, sind z.B. Verbindungen auf der Basis sterisch gehinderter Phenole oder Schwefel oder Phosphor enthaltende Costabilisatoren.

Als derartige phenolische Antioxidationsmittel seien beispielsweise 2,6-Di-tert.-butyl-4-methylphenol, n-Octadecyl-β-(3,5-di-tert.-butyl-4-hydroxyphenyl)-propionat, 1,1,3-Tris-(2-methyl-4-hydroxy-5-tert.-butylphenyl)-butan, 1,3,5-Trimethyl-2,4,6-tris-(3,5-di-tert.-butyl-4-hydroxybenzyl)-benzol, 1,3,5-Tris-(3,5-di-tert.-buty)-4-hydroxybenzyl)-iso-cyanurat, 1,3,5-Tris-[β-(3,5-di-tert.-butyl-4-hydroxyphenyl)-propionylethyl]isocyanurat, 1,3,5-Tris-(2,6-dimethyl-3-hydroxy-4-tert.-butylbenzyl)-isocyanurat und Pentaerythrit-tetrakis-[β-(3,5-di-tert.-butyl-4-hydroxy-phenyl)-propionat] erwähnt.

Als phosphorhaltige Antioxidantien kommen beispielsweise Tris-(nonylphenyl)-phosphit, Distearylpentaerythritdiphosphit, Tris-(2,4-di-tert.-butyl-phenyl)-phosphit, Tris-(2-tert.-butyl-4-methylphenyl)-phosphit, Bis-(2,4-di-tert.-butylphenyl)-pentaerythritdiphosphit und Tetrakis-(2,4-di-tert.-butylphenyl)-4,4'-biphenylendiphosphit in Betracht.

Als Schwefel enthaltende Antioxidationsmittel seien beispielsweise Dilaurylthiodipropionat, Dimyristylthiodipropionat, Distearylthiodipropionat, Pentaerythrittetrakis-(β-laurylthiopropionat) und Pentaerythrittetrakis-(β-hexylthlopropionat) genannt.

Weitere Antioxidatien und Lichtstabilisatoren, die zusammen mit den erfindungsgemäßen Formulierungen verwendet werden können, sind z.B. 2-(2'-Hydroxyphenyl)-benztriazole, 2-Hydroxybenzophenone, Arylester von Hydroxybenzoesäuren, α-Cyanozimtsäurederivate, Benzimidazolcarbonsäureanilide, Nickelverbindungen oder Oxalsäuredianilide.

Eine besonders gute Stabilisierung erhält man, wenn zu den erfindungsgemäßen Formulierungen noch mindestens ein Lichtstabilisator aus der Verbindungsklasse der sterisch gehinderten Amine in üblicher Konzentration zugesetzt wird.

Als sterisch gehinderte Amine kommen z.B. in Betracht: Bis-(2,2,6,6-tetramethylpiperidyl)-sebacat, Bis-(1,2,2,6,6-pentamethylpiperidyl)-sebacat, das Kondensationsprodukt von 1-Hydroxyethyl-2,2,6,6-tetramethyl-4-hydroxypiperidin und Bernsteinsäure, das Kondensationsprodukt von N,N'-Di-(2,2,6,6-Tetramethylpiperidyl)-hexamethylendiamin und 4-tert.-Octylamino-2,6-dichlor-1,3,5-Benzoylbenzoat, Tris-(2,2,6,6-Tetramethylpiperidyl)-nitrilotriacetat, Tetrakis-(2,2,6,6-tetramethyl-4-piperidyl)-1,2,3,4-butantetracarboxylat, 1,1'-(1,2-Ethandiyl)-bis-(3,3,5,5-tetramethylpiperazinon), die Kondensationsprodukte von 4-Amino-2,2,6,6-tetramethylpiperidinen und Tetramethylolacetylendiharnstoffen.

Als Kunststoffe, die durch die erfindungsgemäßen Verbindungen I stabilisiert werden können, seien beispielsweise genannt:
Polymere von Mono- und Diolefinen, wie z.B. Polyethylen niedriger oder hoher Dichte, Polypropylen, lineares Polybuten-1, Polyisopren, Polybutadien sowie Copolymerisate von Mono- oder Diolefinen oder Mischungen der genannten Polymeren;
Copolymerisate von Mono- oder Diolefinen mit anderen Vinylmonomeren, wie z.B. Ethylen-Alkylacrylat-Copolymere, Ethylen-Alkylmethacrylat-Copolymere, EthylenVinylacetat-Copolymere oder Ethylen-Acrylsäure-Copolymere;
Polystyrol und Copolymere von Styrol oder α-Methylstyrol mit Dienen und/oder Acrylderivaten, wie z.B. Styrol-Butadien, Styrol-Acrylnitril (SAN), Styrol-Ethylmethacrylat, Styrol-Butadien-Ethylacrylat, Styrol-Acrylnitril-Methacrylat, Acrylnitril-Butadien-Styrol (ABS) oder Methylmethacrylat-Butadien-Styrol (MBS);
halogenhaltige Polymere, wie z.B. Polyvinylchlorid, Polyvinylfluorid, Polyvinylidenfluorid sowie deren Copolymere;
Polymere, die sich von α,β-ungesättigten Säuren und deren Derivaten ableiten, wie Polyacrylate, Polymethacrylate, Polyacrylamide und Polyacrylnitrile;
Polymere, die sich von ungesättigten Alkoholen und Aminen bzw. von deren Acylderivaten oder Acetalen ableiten, z.B. Polyvinylalkohol und Polyvinylacetat;
Polyurethane, Polyamide, Polyharnstoffe, Polyester, Polycarbonate, Polysulfonate, Polyethersulfone und Polyetherketone.

Weiterhin können mit den erfindungsgemäßen Formulierungen wässrige Dispersionslacke sowie Lacküberzüge stabilisiert werden, z.B. Industrielackierungen. Unter diesen sind Einbrennlackierungen, unter diesen wiederum Fahrzeuglackierungen, vorzugsweise Zweischichtlackierungen, besonders hervorzuheben.

Die Formulierungen können in fester oder flüssiger Form dem Lack zugesetzt werden. Ihre gute Löslichkeit in Lacksystemen ist dabei von besonderem Vorteil.

Auch bei der Verwendung als Stabilisatoren in Lacken können die bereits aufgeführten zusätzlichen Additive, insbesondere Antioxidantien und Lichtstabilisatoren, mitverwendet werden.

Ganz besonders bevorzugt eignen sich die erfindungsgemäßen Lichtschutzmittel Formulierungen auch als photostabile UV-Filter in kosmetischen und dermatologischen Zubereitungen zum Schutz der menschlichen Haut oder menschlicher Haare gegen Sonnenstrahlen aber auch gegen künstliches Licht, welches hohe UV-Anteile aufweist, allein oder zusammen mit für kosmetische oder pharmazeutische Zubereitungen bekannten, im UV-Bereich absorbierenden Verbindungen. Unter organischen Materialien sind im weitesten Sinne somit auch die menschliche Haut und menschliche Haare zu verstehen. Die kosmetischen und pharmazeutischen Zubereitungen als solche werden zugleich natürlich auch stabilisiert, um möglichst lange wirksam zu bleiben.

Demgemäß sind auch Gegenstand der vorliegenden Erfindung Lichtschutzmittel enthaltende kosmetische und pharmazeutische Zubereitungen zum Schutz der menschlichen Haut oder menschlicher Haare gegen UV-Licht im Bereich von 280 bis 400 nm, dadurch gekennzeichnet, dass sie in einem kosmetisch oder pharmazeutisch geeigneten Träger als photostabile UV-Filter wirksame Mengen einer Formulierung des Benzoylbenzoats I in amorpher oder teilamorpher Form - allein oder zusammen mit an sich für kosmetische und pharmazeutische Zubereitungen bekannten, im UV-A- und UV-B-Bereich absorbierenden Verbindungen - enthalten, wobei es sich bei den Formulierungen um die eingangs genannten erfindungsgemäßen wässrigen Dispersionen oder den daraus hergestellten Trockenpulvern handelt.

Die Menge an Benzoylbenzoat I in Form der erfindungsgemäßen Formulierungen, die in den kosmetischen und pharmazeutischen Zubereitungen eingesetzt wird, liegt im Bereich von 0,05 bis 20 Gew.-%, bevorzugt 0,1 bis 10 Gew.-%, besonders bevorzugt im Bereich von 1 bis 7 Gew.-%, bezogen auf die gesamte Menge der kosmetischen und pharmazeutischen Zubereitung.

Die Lichtschutzmittel enthaltenden kosmetischen und pharmazeutischen Zubereitungen sind in der Regel auf der Basis eines Trägers, der mindestens eine Ölphase enthält. Es sind aber auch Zubereitungen allein auf wässriger Basis möglich. Demgemäß kommen Öle, Öl-in-Wasser- und Wasser-in-Öl-Emulsionen, Cremes und Pasten, Lippenschutzstiftmassen oder fettfreie Gele in Betracht.

Als Emulsionen kommen u.a. auch O/W-Makroemulsionen, O/W-Mikroemulsionen oder O/W/O-Emulsionen mit in dispergierter Form vorliegenden aminosubstituierten Hydroxybenzophenonen der Formel I in Frage, wobei die Emulsionen durch Phaseninversionstechnologie, gemäß DE-A-197 26121 erhältlich sind.

Übliche kosmetische Hilfsstoffe, die als Zusätze in Betracht kommen können, sind z.B. Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind u.a. Bienenwachs, Paraffinwachs oder Mikrowachse gegebenenfalls in Kombination mit hydrophilen Wachsen zu nennen. Als Stabilisatoren können Metallsalze von Fettsäuren wie z.B. Magnesium-, Aluminium- und/oder Zinkstearat eingesetzt werden. Geeignete Verdickungsmittel sind beispielsweise vemetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polyacrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösung, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 0,1 Gew.-%, bezogen auf die gesamte Mischung, eingesetzt.

Ein zusätzlicher Gehalt an Antioxidantien ist im allgemeinen bevorzugt. So können als günstige Antioxidantien alle für kosmetische und/oder dermatologische Anwendungen geeigneten oder gebräuchlichen Antioxidantien verwendet werden.

Vorteilhafterweise werden die Antioxidantien gewählt aus der Gruppe, bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Camosin, L-Carnosin und deren Derivate (z.B. Anserin), Carotinoide, Carotine (z.B. β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thiorodoxin, Glutathion, Cystein, Cystin, Cystamin und deren Glycosyl-, N-Acetyl-, Methyl-, Ethyl-, Propyl-, Amyl-, Butyl-, und Lauryl-, Palmitoyl-, Oleyl-, γ-Linoleyl-, Cholesteryl- und Glycerylester) sowie deren Salze, Dilaurylthiodipropionat, Distearylthiodipropioriat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximine, Buthioninsulfone, Penta-, Hexa-, Heptathioninsulfoximin) in sehr geringen verträglichen Dosierungen (z.B. pmol bis ∝mol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und deren Derivate (z.B. Ascorbylpalmitat, Mg-Ascorbylphosphat, Ascorbylacetat), Tocopherol und Derivate (z.B. Vitamin-E-Acetat, Tocotrienol), Vitamin A und Derivate (Vitamin-A-Palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, Trans-Stilbenoxid).

Die Menge der vorgenannten Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Zubereitung.

Sofern Vitamin E und/oder dessen Derivate das oder die Antioxidantien darstellen, ist es vorteilhaft, deren jeweilige Konzentration aus dem Bereich von 0,001 bis 10 Gew.-%, bezogen auf das Gesamtgewicht der Formulierung, zu wählen.

Sofern Vitamin A und/oder dessen Derivate bzw. Carotinoide das oder die Antioxidantien darstellen, ist es vorteilhaft, deren jeweilige Konzentration aus dem Bereich von 0,001 bis 10 Gew.-%, bezogen auf das Gesamtgewicht der Formulierung, zu wählen.

Übliche Ölkomponenten in der Kosmetik sind beispielsweise Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure.

Der Gesamtanteil der Hilfs- und Zusatzstoffe kann 1 bis 80, vorzugsweise 6 bis 40 Gew.-% und der nicht wässrige Anteil ("Aktivsubstanz") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

Solche Sonnenschutzpräparate können demgemäß in flüssiger, pastöser oder fester Form vorliegen, beispielsweise als Wasser-in-Öl-Cremes, Öl-in-Wasser-Cremes und -Lotionen, Aerosol-Schaumcremes, Gele, Öle, Fettstifte, Puder, Sprays oder alkoholisch-wässrige Lotionen.

Schließlich können weitere an sich bekannte im UV-Bereich absorbierenden Substanzen mitverwendet werden, sofern sie im Gesamtsystem der erfindungsgemäß zu verwendenden Kombination aus UV-Filtern stabil sind.

Der größte Teil der Lichtschutzmittel in den zum Schutz der menschlichen Epidermis dienenden kosmetischen und pharmazeutischen Zubereitungen besteht aus Verbindungen die UV-Licht im UV-B-Bereich absorbieren d.h. im Bereich von 280 bis 320 nm. Beispielsweise beträgt der Anteil der erfindungsgemäß zu verwendenden UV-A-Absorber 10 bis 90 Gew.-%, bevorzugt 20 bis 50 Gew.-% bezogen auf die Gesamtmenge von UV-B und UV-A absorbierenden Substanzen.

Als UV-Filtersubstanzen, die in Kombination mit den erfindungsgemäß zu verwendenden Formulierungen angewandt werden, kommen beliebige UV-A- und UV-B-Filtersubstanzen in Betracht. Beispielsweise sind zu nennen:

| Nr. | Stoff | CAS-Nr. (=Säure) |
|---|---|---|
| 1 | 4-Aminobenzoesäure | 150-13-0 |
| 2 | 3-(4'-Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxy-ethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfonsäure-(Sulisobenzonum) und das Natriumsalz | 4065-45-6 |
| 13 | 3-(4'-Sulfobenzyliden)-bornan-2-on und Salze | 58030-58-6 |
| 14 | 3-Benzylidenbornan-2-on | 16087-24-8 |
| 15 | 1-(4'-Isopropylphenyl)-3-phenylpropan-1,3-dion | 63260-25-9 |
| 16 | 4-lsopropylbenzylsalicylat | 94134-93-7 |
| 17 | 3-Imidazol-4-yl-acrylsäure und ihr Ethylester | 104-98-3 |
| 18 | 2-Cyano-3,3-diphenylacrylsäureethylester | 5232-99-5 |
| 19 | 2-Cyano-3,3-diphenylacrylsäure-2'-ethylhexylester | 6197-30-4 |
| 20 | Menthyl-o-aminobenzoat oder: 5-Methyl-2-(1-methylethyl)-2-aminobenzoat | 134-09-8 |
| 21 | Glyceryl p-aminobenzoat oder: 4-Aminobenzoesäure-1-glyceryl-ester | 136-44-7 |
| 22 | 2,2'-Dihydroxy-4-methoxybenzophenon (Dioxybenzone) | 131-53-3 |
| 23 | 2-Hydroxy-4-methoxy-4-methylbenzophenon (Mexenon) | 1641-17-4 |
| 24 | Triethanolamin Salicylat | 2174-16-5 |
| 25 | Dimethoxyphenylglyoxalsäure oder: 3,4-dimethoxy-phenyl-glyoxal-saures Natrium | 4732-70-1 |
| 26 | 3-(4'Sulfobenzyliden)-bornan-2-on und seine Salze | 56039-58-8 |
| 27 | 4-tert.-Butyi-4'-methoxy-dibenzoylmethan | 70356-09-1 |
| 28 | 2,2',4,4'-Tetrahydroxybenzophenon | 131-55-5 |
| 29 | 2,2'-Methylen-bis-[6-(2H-benzotriazol-2-yl)-4-(1,1,3,3,-tetramethylbutyl)phenol] | 103597-45-1 |
| 30 | 2,2'-(1,4-Phenylen)-bis-1H-benzimidazol-4,6-disulfonsäure, Na-Salz | 180898-37-7 |
| 31 | 2,4-bis-[4-(2-Ethylhexyloxy)-2-hydroxy]phenyl-6-(4-methoxyphenyl)-(1,3,5)-triazin | 187393-00-6 |
| 32 | 3-(4-Methylbenzyliden)-campher | 36861-47-9 |
| 33 | 4-Bis(polyethoxy)paraaminobenzoesäurepolyethoxyethylester | 113010-52-9 |
| 34 | 2,4-Dihydroxybenzophenon | 131-56-6 |
| 35 | 2,2'-Dihydroxy-4,4'-dimethoxybenzophenon-5,5'-dinatriumsulfonat | 3121-60-6 |
| 36 | 2,4,6-Trianilin-(p-carbo-2'-ethylhexyl-1'-oxy)-1,3,5-triazin | 88122-99-0 |

Auch polymere oder polymergebundene Filtersubstanzen können erfindungsgemäß verwendet werden.

Die erfindungsgemäßen kosmetischen und dermatologischen Zubereitungen können vorteilhafterweise außerdem anorganische Pigmente auf Basis von Metalloxiden und/oder anderen in Wasser schwerlöslichen oder unlöslichen Metallverbindungen, insbesondere der Oxide des Titans (TiO₂), Zinks (ZnO), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden enthalten. Besonders bevorzugt handelt es sich um Pigmente auf der Basis TiO₂ und ZnO.

Die anorganischen Pigmente können dabei in hydrophober Form vorliegen, d.h. dass sie oberflächlich wasserabweisend behandelt sind. Diese Oberflächenbehandlung kann darin bestehen, dass die Pigmente nach an sich bekannter Weise, wie in DE-A-33 14 742 beschrieben, mit einer dünnen hydrophoben Schicht versehen sind.

Zum Schutz menschlicher Haare vor UV-Strahlen können die erfindungsgemäßen Lichtschutzmittel-Formulierungen in Shampoos, Lotionen, Gelen, Haarsprays, Haarfärbemittel, Aerosol-Schaumcremes oder Emulsionen in Konzentrationen von 0,1 bis 10 Gew.-%, bevorzugt 1 bis 7 Gew.-% eingearbeitet werden. Die jeweiligen Formulierungen können dabei u.a. zum Waschen, Färben sowie zum Frisieren der Haare verwendet werden.

Die erfindungsgemäß zu verwendenden Formulierungen zeichnen sich in der Regel durch ein besonders hohes Absorptionsvermögen im Bereich der UV-A-Strahlung mit scharfer Bandenstruktur aus. Weiterhin sind sie gut in kosmetischen Ölen löslich und lassen sich leicht in kosmetische Formulierungen einarbeiten. Die mit den Formulierungen hergestellten Emulsionen zeichnen sich besonders durch ihre hohe Stabilität, die Formulierungen I selber durch ihre hohe Photostabilität aus, und die damit hergestellten Zubereitungen durch ihr angenehmes Hautgefühl aus.

Die UV-Filterwirkung der erfindungsgemäßen Formulierungen kann auch zur Stabilisierung von Wirk- und Hilfsstoffen in kosmetischen und pharmazeutischen Formulierungen ausgenutzt werden.

Die erfindungsgemäßen Zubereitungen zeichnen sich durch ein besonders hohes Absorptionsvermögen im Bereich der UV-A-Strahlung mit scharfer Bandenstruktur und hohen Lichtschutzfaktoren aus.

Insbesondere der hohe Lichtschutzfaktor der Zubereitungen, der bereits bei niedrigen Konzentrationen an Benzoylbenzoat I gemessen wurde, war überraschend.

Ferner haben die erfindungsgemäßen Zubereitungen den Vorteil einer verbesserten Kaltwasserdispergierbarkeit

Die folgenden Beispiele sollen die vorliegende Erfindung verdeutlichen, ohne sie einzuschränken.

### Beispiel 1

Herstellung eines Uvinul^{®} A Plus-haltigen Trockenpulvers mit einem Wirkstoffgehalt von 20 Gew.-%
a) Herstellung der wässrigen Dispersion
   12,5 g Uvinul^{®} A Plus wurden in 216 g Isopropanol/Wasser (9:1) bei Raumtempertur molekular-dispers gelöst. Zur Ausfällung des Uvinul^{®} A Plus in kolloiddisperser Form wurde die Lösung bei 240°C einer Mischkammer zugeführt. Dort erfolgte die Vermischung mit einer wässrigen Lösung von 22,5 g HiCap in 1477,5 ml VE-Wasser. Der gesamte Prozess erfolgte unter Druckbegrenzung auf 40 bar, um eine Verdampfung des Lösemittels zu verhindern. Nach dem Mischen wurde eine kolloiddisperse Uvinul^{®} A Plus-Dispersion mit einem weiß-trüben Farbton erhalten.
   Mittels Fraunhofer-Beugung wurde der Mittelwert der Volumenverteilung zu D (4,3) = 0,20 µm bei einem Feinanteil der Verteilung 100 % < 1,22 µm bestimmt.
b) Herstellung eines Uvinul^{®} A Plus-haltigen wässrigen Trockenpulvers
   Nach Sprühtrocknung der Dispersion erhielt man ein Trockenpulver mit einem Wirkstoffgehalt von 21,20 Gew.-% Uvinul^{®} A Plus (Gehaltsbestimmung erfolgte UV/VIS-spektroskopisch). Das Trockenpulver ließ sich erneut in VE-Wasser unter Bildung einer weiß-trüben Dispersion (Hydrosol) redispergieren.
   Mittels Fraunhofer-Beugung wurde der Mittelwert der Volumenverteilung in der Re-Dispersion bestimmt zu D (4,3) = 0,45 µm bei einem Feinanteil der Verteilung von 96,73 % < 1,22 µm.

### Zubereitungen

### Beispiel 2: Zusammensetzung für die Lippenpflege

### Massengehalt (Gew.-%)

| | |
|---|---|
| ad 100 | Eucerinum anhydricum |
| 10,00 | Glycerin |
| 10,00 | Titanium Dioxid, mikronisiert |
| 5,00 | Uvinul^{®} A Plus Trockenpulver aus Beispiel 1 |
| 8,00 | Octyl Methoxycinnamat |
| 5,00 | Zink Oxid |
| 4,00 | Castoröl |
| 4,00 | Pentaerythrithil Stearat/caprat/Caprylat Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

### Beispiel 3: Zusammensetzung für Sunblocker mit Mikropigmenten

### Massengehalt (Gew.-%)

| | |
|---|---|
| ad 100 | Wasser |
| 10,00 | Octyl Methoxcinnamat |
| 6,00 | PEG-7-Hydrogenated Castor Öl |
| 6,00 | Titanium Dioxid, mikronisiert |
| 5,00 | Uvinul^{®} A Plus Trockenpolver aus Beispiel 1 |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

### Beispiel 4: Fettfreies Gel

### Massengehalt (Gew.-%)

| | |
|---|---|
| ad 100 | Wasser |
| 8,00 | Octyl Methoxycinnamat |
| 7,00 | Titanium Dioxid, mikronisiert |
| 5,00 | Uvinul^{®} A Plus Trockenpulver aus Beispiel 1 |
| 5,00 | Glycerin |
| 5,00 | PEG-25 PABA |
| 1,00 | 4-Methylbenzyliden Campher |
| 0,40 | Acrylate C₁₀-C₃₀ Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 5: Sonnencreme (LSF 20)

### Massengehalt (Gew.-%)

| | |
|---|---|
| ad 100 | Wasser |
| 8,00 | Octyl Methoxycinnamat |
| 8,00 | Titanium Dioxid, mikronisiert |
| 6,00 | PEG-7-Hydrogenated Castor Öl |
| 5,00 | Uvinul^{®} A Plus Trockenpulver aus Beispiel 1 |
| 6,00 | Mineral Öl |
| 5,00 | Isopropyl Palmitat |
| 0,30 | lmidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 6: Sonnencreme wasserfest

### Massengehalt (Gew.-%)

| | |
|---|---|
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 5,00 | Uvinul^{®} A Plus Trockenpulver aus Beispiel 1 |
| 4,00 | Glycerin |
| 3,00 | Jojoba Öl |
| 2,00 | 4-Methylbenzyliden Campher |
| 2,00 | Titanium Dioxid, mikronisiert |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

### Beispiel 7: Sonnenmilch (LSF 6)

### Massengehalt (Gew.-%)

| | |
|---|---|
| ad 100 | Wasser |
| 10,00 | Mineral Öl |
| 6,00 | PEG-7-Hydrogenated Castor Öl |
| 5,00 | Isopropyl Palmitat |
| 3,50 | Octyl Methoxycinnamat |
| 5,00 | Uvinul^{®} A Plus Trockenpulver aus Beispiel 1 |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 3,00 | Glycerin |
| 0,25 | Methylparaben |
| 0,15 | Propylparaben |
| 0,05 | Tocopherol |

### Beispiel 8: Tageslotion mit UV-Schutz

### Massengehalt (Gew.-%)

| | |
|---|---|
| ad 100 | Wasser |
| 2,00 | Cetearyl Alkohol |
| 1,00 | Glycerinmonostearat |
| 2,00 | Vaselin |
| 7,50 | Octyl Methoxycinnamat |
| 4,00 | Octyl Salicylat |
| 3,00 | Uvinul^{®} A Plus Trockenpulver aus Beispiel 1 |
| 1,50 | 4-tert.-Butyl-4'-methoxy-dibenzoylmethan |
| 0,50 | Dimethicon |
| 5,00 | Propylen Glycol |
| 0,20 | EDTA |
| 0,20 | Carbomer |
| 5,00 | C₁₂-C₁₅ Alkyl Benzoat |
| 0,27 | Triethanolamin |
| 1,00 | Tocopheryl Acetat |
| q.s. | Fragrance |

### Beispiel 9: Tagescreme mit UV-Schutz

### Massengehalt (Gew.-%)

| | |
|---|---|
| ad 100 | Wasser |
| 2,00 | Cetearyl Alkohol |
| 2,00 | Cetyl Alkohol |
| 1,00 | Glycerinmonostearat |
| 2,00 | Vaselin |
| 7,50 | Octyl Methoxycinnamat |
| 4,00 | Octyl Salicylat |
| 3,00 | Uvinul^{®} A Plus Trockenpulver aus Beispiel 1 |
| 1,50 | 4-tert.-Butyl-4'-methoxy-dibenzoylmethan |
| 4,00 | Propylen Glycol |
| 0,20 | EDTA |
| 0,20 | Carbomer |
| 0,20 | Xanthan |
| 0,20 | C₁₀-C₃₀ Alkyl Acrylate Crosspolymer |
| 5,00 | C₁₂-C₁₅ Alkyl Benzoat |
| 0,54 | Triethanolamin |
| 1,00 | Tocopheryl Acetat |
| q.s. | Fragrance |
| q.s. | Konservierungsmittel |

### Beispiel 10: Flüssiges Make Up

### Massengehalt (Gew.-%)

| | |
|---|---|
| ad 100 | Wasser |
| 2,00 | Cetearyl Alkohol |
| 2,00 | Ceteareth 25 |
| 6,00 | Glycerinmonostearat |
| 1,00 | Cetylalkohol |
| 8,00 | Paraffinöl |
| 7,00 | CetearylOctanoat |
| 0,2 | Dimethicon |
| 3,00 | Propylen Glycol |
| 1,00 | Panthenol |
| 3,00 | Uvinul^{®} A Plus Trockenpulver aus Beispiel 1 |
| 1,50 | 4-tert.-Butyl-4'-methoxy-dibenzoylmethan |
| 3,50 | Octyl Methoxycinnamat |
| 0,1 | Bisabolol |
| 5,70 | Titandioxid |
| 1,10 | Eisenoxid |
| q.s. | Fragrance |

### Beispiel 11: Haargel mit Sonnenschutz

### Massengehalt (Gew.-%)

| | |
|---|---|
| ad 100 | Wasser |
| 1,20 | Carbomer |
| 0,50 | Hydroxyethyl Cellulose |
| 4,00 | Triethanolamin |
| 0,70 | PEG-40 Hydrogenated Castor oil |
| 1,50 | Uvinul^{®} A Plus Trockenpulver aus Beispiel 1 |
| 0,70 | 4-tert.-Butyl-4'-methoxy-dibenzoylmethan |
| 2,80 | Octyl Methoxycinnamat |
| 5,00 | Propylen Glycol |
| 0,01 | EDTA |
| q.s. | Fragrance |
| q.s. | Sicovit Patentblau 85 E 131 |

## Patentansprüche

1. Verfahren zur Herstellung pulverförmiger Zubereitungen, enthaltend Diethylamino-hydroxybenzoyl-hexyl-benzoat der Formel I 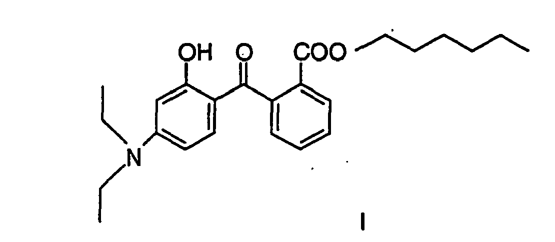 durch
a) Dispergieren des Benzoylbenzoats I in einer wässrigen molekulardispersen oder kolloiddispersen Lösung eines Schutzkolloids und
b) Überführung der gebildeten Dispersion in ein Trockenpulver durch Abtrennung des Wassers und gegebenenfalls zusätzlich verwendeter Lösungsmittel und Trocknung,
**dadurch gekennzeichnet, dass** man als Schutzkolloid im Verfahrensschritt a) modifizierte Stärke verwendet.

2. Verfahren nach Anspruch 1, **dadurch gekennzeichnet, dass** das Dispergieren in der Stufe a) folgende Schritte umfasst:
a₁) Lösen des Benzoylbenzoats I in einem oder mehreren mit Wasser mischbaren; organischen Lösungsmittel(n) oder in einer Mischung aus Wasser und einem oder mehreren mit Wasser mischbaren, organischen Lösungsmittel(n) oder
a₂) Lösen des Benzoylbenzoats I in einem oder mehreren mit Wasser nicht mischbaren, organischen Lösungsmittel(n) und
a₃) Mischen der nach a₁) oder a₂) erhaltenen Lösung mit einer wässrigen molekulardispersen oder kolloiddispersen Lösung von modifizierter Stärke, wobei die hydrophobe Phase der Verbindung I als nanodisperse Phase entsteht.

3. Verfahren nach einem der Ansprüche 1 oder 2, **dadurch gekennzeichnet, dass** die Trocknung im Verfahrensschritt b) in Gegenwart eines Überzugsmaterials erfolgt.

4. Verfahren nach einem der Ansprüche 1 bis 3, **dadurch gekennzeichnet, dass** es sich beim Dispergierschritt a) um die Herstellung einer Suspension des Benzoylbenzoats I in einer wässrigen molekulardispersen oder kolloiddispersen Lösung von modifizierter Stärke handelt.

5. Verfahren nach Anspruch 4, **dadurch gekennzeichnet, dass** man die im Verfahrensschritt a) hergestellte Suspension vor der Überführung in ein Trockenpulver mahlt.

6. Verfahren nach Anspruch 4, **dadurch gekennzeichnet, dass** man
a₁) das Benzoylbenzoat I in Aceton oder Isopropanol oder einer Mischung aus Wasser und Aceton oder Wasser und Isopropanol bei Temperaturen im Bereich von 50 bis 240°C löst,
a₃) die erhaltene Lösung mit einer wässrigen molekulardispersen oder kolloiddispersen Lösung von modifizierter Stärke bei Temperaturen im Bereich von 25 bis 120°C mischt und
b) die gebildete Suspension nach Abtrennung des organischen Lösungsmittels sprühtrocknet.

7. Verfahren nach einem der Ansprüche 1 bis 3, **dadurch gekennzeichnet, dass** es sich beim Dispergierschritt a) um die Herstellung einer Emulsion des Benzoylbenzoats I in einer wässrigen molekulardispersen oder kolloiddispersen Lösung von modifizierter Stärke handelt.

8. Verfahren nach einem der Ansprüche 1 bis 7, **dadurch gekennzeichnet, dass** man als Schutzkolloid Octenyl-succinat-Stärke verwendet.

9. Diethylamino-hydroxybenzoyl-hexyl-benzoat-haltige pulverförmige Zubereitungen, erhältlich nach einem Verfahren, definiert gemäß einem der Ansprüche 1 bis 8.

10. Zubereitungen nach Anspruch 9 mit einem Gehalt an Benzoylbenzoat I von 0,1 bis 70 Gew.-%.

11. Verwendung der Benzoylbenzoat-haltigen Pulver, definiert gemäß einem der Ansprüche 9 oder 10, als photostabile UV-Filter in kosmetischen und dermatologischen Zubereitungen.

## Claims

1. A method of producing a powdered preparation comprising diethylamino hydroxybenzoyl hexyl benzoate of the formula I 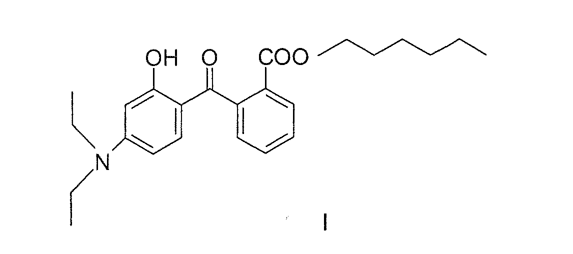 by
a) dispersing the benzoyl benzoate I in an aqueous molecularly disperse or colloidally disperse solution of a protective colloid and
b) converting the dispersion obtained into a dry powder by removing the water and, if appropriate, additionally used solvents, and drying,
wherein the protective colloid used in process step a) is modified starch.

2. The method according to claim 1, wherein the dispersion in stage a) comprises the following steps:
a₁) dissolving the benzoyl benzoate I in one or more water-miscible organic solvent(s) or in a mixture of water and one or more water-miscible organic solvent(s) or
a₂) dissolving the benzoyl benzoate I in one or more water-immiscible organic solvent(s) and
a₃) mixing the solution obtained after a₁) or a₂) with an aqueous molecularly disperse or colloidally disperse solution of modified starch, where the hydrophobic phase of the compound I is formed as nanodisperse phase.

3. The method according to one of claims 1 or 2, wherein the drying in process step b) is carried out in the presence of a coating material.

4. The method according to one of claims 1 to 3, wherein dispersion step a) is the preparation of a suspension of the benzoyl benzoate I in an aqueous molecularly disperse or colloidally disperse solution of modified starch.

5. The method according to claim 4, wherein the suspension prepared in process step a) is ground before being converted into a dry powder.

6. The method according to claim 4, wherein
a₁) the benzoyl benzoate I is dissolved in acetone or isopropanol or a mixture of water and acetone or water and isopropanol at temperatures in the range from 50 to 240°C,
a₃) the solution obtained is mixed with an aqueous molecularly disperse or colloidally disperse solution of modified starch at temperatures in the range from 25 to 120°C and
b) the suspension formed is spray-dried after removing the organic solvent.

7. The method according to one of claims 1 to 3, wherein dispersion step a) is the preparation of an emulsion of the benzoyl benzoate I in an aqueous molecularly disperse or colloidally disperse solution of modified starch.

8. The method according to one of claims 1 to 7, wherein the protective colloid used is octenyl succinate starch.

9. A powdered preparation containing diethylamino hydroxybenzoyl hexyl benzoate, obtainable by a method defined according to one of claims 1 to 8.

10. The preparation according to claim 9 with a content of benzoyl benzoate I of from 0.1 to 70% by weight.

11. The use of the benzoyl-benzoate-containing powder defined according to one of claims 9 or 10 as a photostable LTV filter in cosmetic and dermatological preparations.

## Revendications

1. Procédé de préparation de compositions pulvérulentes, contenant du benzoate de diéthylaminohydroxybenzoylhexyle de formule I : 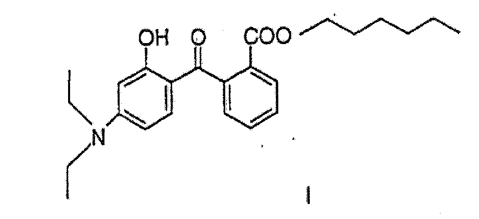 par
a) dispersion du benzoate de benzoyle I dans une solution aqueuse à dispersion moléculaire ou colloïdale d'un colloïde de protection, et
b) conversion de la dispersion formée en une poudre sèche par isolement de l'eau et des solvants éventuellement utilisés en supplément et séchage,
**caractérisé en ce qu'**on utilise de l'amidon modifié comme colloïde de protection dans l'étape a) du procédé.

2. Procédé suivant la revendication 1, **caractérisé en ce que** la dispersion de l'étape a) comporte les phases suivantes :
a₁) dissolution du benzoate de benzoyle I dans un ou plusieurs solvants organiques, miscibles à l'eau, ou dans un mélange d'eau et d'un ou de plusieurs solvants organiques, miscibles à l'eau, ou
a₂) dissolution du benzoate de benzoyle I dans un ou plusieurs solvants organiques, non miscibles à l'eau, et
a₃) mélange de la solution obtenue selon a₁) ou a₂) avec une solution aqueuse à dispersion moléculaire ou colloïdale d'amidon modifié, la phase hydrophobe du composé I étant obtenue sous la forme d'une phase nanodispersée.

3. Procédé suivant l'une des revendications 1 et 2, **caractérisé en ce que** le séchage de l'étape b) du procédé a lieu en présence d'une matière de revêtement.

4. Procédé suivant l'une des revendications 1 à 3, **caractérisé en ce que**, en ce qui concerne l'étape de dispersion a), il s'agit de la préparation d'une suspension du benzoate de benzoyle I dans une solution aqueuse à dispersion moléculaire ou colloïdale d'amidon modifié.

5. Procédé suivant la revendication 4, **caractérisé en ce qu'**on broie la suspension préparée dans l'étape a) du procédé avant la conversion en une poudre sèche.

6. Procédé suivant la revendication 4, **caractérisé en ce que**
a₁) on dissout le benzoate de benzoyle I dans de l'acétone ou de l'isopropanol ou un mélange d'eau et d'acétone ou d'eau et d'isopropanol à des températures de l'ordre de 50 à 240°C,
a₃) on mélange la solution obtenue avec une solution aqueuse à dispersion moléculaire ou colloïdale d'amidon modifié à des températures de l'ordre de 25 à 120°C, et
b) on sèche par pulvérisation la suspension formée, après isolement du solvant organique.

7. Procédé suivant l'une des revendications 1 à 3, **caractérisé en ce que**, pour ce qui concerne l'étape de dispersion a), il s'agit de la préparation d'une émulsion du benzoate de benzoyle I dans une solution aqueuse à dispersion moléculaire ou colloïdale d'amidon modifié.

8. Procédé suivant l'une des revendications 1 à 7, **caractérisé en ce qu'**on utilise, comme colloïde de protection, de l'octényl-succinate-amidon.

9. Compositions pulvérulentes contenant du benzoate de diéthylaminohydroxybenzoylhexyle, que l'on peut obtenir suivant un procédé défini conformément à une des revendications 1 à 8.

10. Compositions suivant la revendication 9, avec une teneur en benzoate de benzoyle I de 0,1 à 70 % en poids.

11. Utilisation de la poudre contenant du benzoate de benzoyle, définie conformément à une des revendications 9 ou 10, comme filtre U.V. photostable dans des compositions cosmétiques et dermatologiques.
